# EUROPEAN PATENT SPECIFICATION

(11) **EP 3 423 567 B1**
(45) Date of publication and mention of the grant of the patent: **03.05.2023**
(21) Application number: 17760841.1
(22) Date of filing: 02.03.2017
(51) Int. Cl.: C12N 5/071, C12N 13/00, A61K 35/12, A61L 26/00, A61L 27/38

(54) **ENHANCED MULTIPOTENT CELLS AND MICROVASCULAR TISSUE AND METHODS OF USE THEREOF**
VERBESSERTE MULTIPOTENTE ZELLEN UND MIKROVASKULÄRES GEWEBE UND VERFAHREN ZUR VERWENDUNG DAVON
CELLULES MULTIPOTENTES ET TISSU MICROVASCULAIRE AMÉLIORÉS ET LEURS PROCÉDÉS D'UTILISATION

(30) Priority: 02.03.2016 US 201662302669 P
(43) Date of publication of application: 09.01.2019
(73) Proprietor: Microvascular Tissues, Inc., San Diego, California 92121 (US)
(72) Inventor: PETERSON, Dale R., Gilbert, AZ 85297 (US); MATTERN, Ralph-Heiko, Ramona California 92065 (US); ARM, Douglas M., Carlsbad California 92009 (US); PICKETT, Lael J., White Bear Lake Minnesota 55110 (US); GONG, Glen, San Carlos California 94070 (US); OHASHI, Kevin L., Jamaica Plain Massachusetts 02130 (US)
(74) Representative: Schiweck Weinzierl Koch Patentanwälte Partnerschaft mbB
(86) International application number: PCT/US2017/020492
(87) International publication number: WO 2017/151950

(56) References cited:
- WO-A1-2012/154301
- US-A1- 2013 243 739
- US-A1- 2015 231 183
- YEW, TU-LAI ET AL.: 'Enhancement of wound healing by human multipotent stromal cell conditioned medium: the paracrine factors and p38 MAPK activa- tion' CELL TRANSPLANTATION vol. 20, 22 December 2010, pages 693 - 706, XP055414306
- ROSOVA, IVANA ET AL.: 'Hypoxic preconditioning results in increased motility and improved therapeutic potential of human mesenchymal stem cells' STEM CELLS vol. 26, 2008, pages 2173 - 2182, XP002643662
- WANG, EN-TONG ET AL.: 'Regulation of tissue repair and regeneration by electric fields' CHINESE JOURNAL OF TRAUMATOLOGY vol. 13, no. 1, 2010, pages 55 - 61, XP026899172

## Description

### CROSS-REFERENCE TO RELATED APPLICATIONS

This application claims priority under 35 U.S.C. § 119(e) to U.S. Application No. 62/302,669, filed March 2, 2016.

### BACKGROUND OF THE INVENTION

The stem cell field has been advancing rapidly for decades. ED Thomas was transplanting patients with hematopoietic stem cells from bone marrow in the 1950's. Friedenstein was the first to discover that there were also mesenchymal stem cells (MSC) in bone marrow. He worked on the topic for many years before publishing in the western literature (Friedenstein AJ, et al., Fibroblast precursors in normal and irradiated mouse hematopoietic organs. Exp Hematol. 1976 Sep; 4(5):267-74). Caplan showed that one could obtain relatively pure populations of mesenchymal stem cells from bone marrow and periosteum by selective adsorption onto plastic and subsequent culture under selective conditions (AI Caplan et al., Mesenchymal stem cells. J Orthop Res. 1991 Sep; 9(5):641-50). During the 1990's, many different groups showed that stem or progenitor cells could be isolated in a similar manner from a wide variety of tissues such as skin, intestine, hair follicles, muscle, liver, brain, placenta, and teeth. Yuan-Di Halvorsen was the first to show that adipose tissue contained cells that could be cultured and differentiated to multiple phenotypes (WO/2001/062901). Most of the literature focuses on identifying and characterizing stem cells and controlling their differentiation to various cell types.

Despite 30 years of work by many companies, no commercial products using MSCs have been able to pass Food and Drug Administration (FDA) requirements. There have been commercial products using stem cells, but they have not been cultured. Products that use fresh bone marrow have been sold since the 1980's (*e.g.,* Collagraft^{®} and Cellect^{™}). DR Peterson was the first to show that fresh cells from adipose and other microvascular tissues could be used for regenerating orthopedic tissues (US Patent No. 6,200,606). Others have since shown that these cells can be used for treating hearts, fistulas, wounds, kidneys, chronic ischemia, cartilage defects, and many other conditions. Historically, these cells were always used in autologous or syngeneic settings.

Osiris Therapeutics, Inc. showed 20 years ago that culturing MSC changes their immunogenicity. The culture process induces the cells to change their surface markers. When these cells are implanted into another animal, they are not usually rejected immediately and, in fact, appear to modulate the host's immune system to prevent rejection. Despite their ability to prevent rejection, MSCs are quickly eliminated in the host's body. Often less than 1% of the injected cells are found three days following implantation. Despite a tremendous amount of work, there is still no proof or even consensus on why the cells die or how they produce beneficial results.

Accordingly, there is a need in the art for methods of enhancing the activity of multipotent cell preparations and uses for such enhanced cell preparations.

### SUMMARY OF THE INVENTION

Recent advances have led to a new approach for using stem cells, multipotent cells, and isolated microvascular tissue. Studies and clinical use have shown that these cells and tissue may be dried and even terminally sterilized yet still retain therapeutic value. Furthermore, the preparation may be used in allogeneic or even xenogeneic subjects. Some additional advantages of the composition include: (1) reduced immune response and reduced likelihood of rejection; (2) anti-inflammatory activity; (3) vascularization activity; (4) sterility; (5) convenience of room temperature storage for extended periods in a form ready for immediate use. Methods of making and using processed or cryopreserved microvascular tissue are described in US Patent No. 9,044,430, US Patent Publication 2015/0231183, and US Patent Publication 2016/0015859.

The present disclosure provides compositions comprising multipotent cells with enhanced bioactivity, and methods for their preparation. The compositions are disclosed for use in treating or preventing a number of different diseases and conditions.

Embodiments of the present invention comprise isolated multipotent cells, processed microvascular tissue, a cell membrane obtained from or derived from said cells or tissue, wherein the cells, tissue, or membrane have vascularization or anti-inflammatory activity. Embodiments of the present invention comprise compositions that have been exposed to treatments that typically reduce potency of cells or proteins but surprisingly enhance bioactivity of the cells or tissue. The treatments include irradiation at the minimum threshold dose of 40kGy. The treatments can further include storage at room temperature, ultrasonic agitation and fluid shear force. In particular embodiments, the composition is dried or lyophilized. In related embodiments, the composition, including the sterilized, dried, or lyophilized, composition, retains measurable bioactivity. In certain embodiments, the composition comprises an excipient.

The compositions disclosed herein can further comprise an implantable scaffold or matrix, which may be, e.g., a bone-derived implant, a scaffold, a porous resorbable polymer, a hydrogel, a putty comprising tissue product, or a suture. Cells, tissue or cell membrane are present on a bone, tendon, or dermal facing surface of said implantable scaffold or matrix.

Compositions can be formulated for intravenous administration. However, other routes of administration may be used in additional embodiments, including direct administration (either to a tissue surface or by direct injection), intra-arterial administration, systemic administration and the like.

The cells or tissue are obtained from a mammalian donor, optionally a human. In certain embodiments, the cells comprise stem cells or progenitor cells.

The present invention provides methods of preparing a composition comprising processed microvascular tissue, wherein said composition has enhanced bioactivity, said method comprising: dissociating a microvascular tissue sample obtained from a donor mammal to produce a composition comprising dissociated microvascular tissue; removing one or more tissue components from the composition comprising dissociated microvascular tissue; optionally drying or lyophilizing the composition before or after sterilization; and treating before or after the optional drying or lyophilizing wherein the composition has increased potency after treatment. In particular embodiments, the cells or composition are not cultured. In particular embodiments, the treatment step comprises exposure to irradiation at the minimum threshold dose of 40kGy, and can comprise the step of heat, osmotic shock, pH excursions, ultrasonic agitation, filtration, density gradient separation, hypoxia, reactive oxygen species, and/or electromagnetic fields. In certain embodiments, the dissociation or removing comprises ultrasonic agitation, filtration, or use of a density gradient. In particular embodiments, the microvascular tissue is adipose-derived tissue, and said ultrasonic agitation, filtration or use of a density gradient removes adipocytes from the composition.

Described herein, but not claimed is a method of treating injury or disease and/or promoting wound healing, in a mammal, comprising providing to a mammal a composition of the present invention or a composition prepared according to a method of the present invention. The composition can be applied topically. The composition can be surgically implanted into the mammal. The composition can be implanted or injected within or adjacent to a site of injury or disease in said mammal. The composition can be provided to a mammal intravenously.

The disease or condition to be treated is a tissue or organ disease. The tissue may be any such as but not limited to epithelial, connective, muscular, adipose, or nerve. The organ may be any such as but not limited organs of digestion, respiration, excretion, endocrine, circulation, sensory, reproduction, and nervous. These include but are not limited to stomach, liver, small intestine, large intestine, rectum, anus, lungs, nose, bronchi, kidneys, urinary bladder, urethra, pituitary gland, adrenal, thyroid, pancreas, parathyroid, prostate, heart, blood vessels, spleen, skin, tongue, eyes, ears, uterus, testis, penis, ovaries, mammary glands, brain and spinal cord. In certain embodiments, the disease or condition is hair loss, bursitis, cleft palate, osteomyelitis, or peripheral neuropathy. In other embodiments, the disease or condition is joint hypermobility/Ehlers-Danlos syndrome, complex regional pain syndrome (CRPS), myofascial pain syndrome, ailing dental implant, Peyronie's disease, Crohn's disease, auto-immune hepatitis, interstitial cystitis, or lichen sclerosis.

The injury can be present in a soft tissue. The injury can be present in a tendon, a ligament, skin, a bone, cartilage, a disc, or microvascular tissue. The injury or disease can be an ischemic injury, a reperfusion injury, a microvascular injury, or inflammation. The disease can be tendinopathy. The disease can be arthritis, such as e.g., osteoarthritis or rheumatoid arthritis. The disease can be plantar fasciitis. The disease can be temporomandibular joint disorder. The method can comprise administration of a composition to treat or prevent scars.

### BRIEF DESCRIPTION OF THE DRAWINGS

**Figure 1** shows the effects of radiation on bioactivity of processed microvascular tissue as measured by HUVEC migration.
**Figure 2** shows the effect of irradiation on cell count.
**Figure 3** shows the effect of storage time on bioactivity of processed microvascular tissue as measured by HUVEC migration.
**Figure 4** shows the effect of storage temperature on bioactivity of processed microvascular tissue as measured by HUVEC migration.
**Figure 5** is a pre-injection sagittal view of a focal linear intra-substance patellar tendon tear.
**Figure 6** is a 28-day post injection sagittal view of the same patellar tendon tear.
**Figure 7** is an image of a large diabetic foot ulcer on plantar surface.
**Figure 8** is the same ulcer one week after treatment with mVASC.
**Figure 9** shows the activity of films of microvascular tissue compared to enzymatically treated microvascular tissue in cell proliferation assays.

### DETAILED DESCRIPTION OF THE INVENTION

Unless otherwise indicated, the practice of the disclosure involves conventional techniques commonly used in molecular biology, medicine, surgery, mammalian physiology, protein purification, protein engineering, cell biology, and tissue engineering fields, which are within the skill of the art. Such techniques are known to those of skill in the art, and are described in numerous standard texts and reference works.

Unless defined otherwise herein, all technical and scientific terms used herein have the same meaning as commonly understood by one of ordinary skill in the art to which this disclosure belongs. Various scientific dictionaries that include the terms included herein are well known and available to those in the art. Although any methods and materials similar or equivalent to those described herein find use in the practice or testing of the disclosure, some preferred methods and materials are described. Accordingly, the terms defined immediately below are more fully described by reference to the specification as a whole. It is to be understood that this disclosure is not limited to the particular methodology, protocols, and reagents described, as these may vary, depending upon the context in which they are used by those of skill in the art.

### DEFINITIONS

As used herein, the singular terms "a", "an," and "the" include the plural reference unless the context clearly indicates otherwise.

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The headings provided herein are not limitations of the various aspects or embodiments of the invention that can be had by reference to the specification as a whole.

The term "invention" or "present invention" as used herein is a non-limiting term and is not intended to refer to any single embodiment of the particular invention but encompasses all possible embodiments as described in the specification and the claims.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length. In any embodiment discussed in the context of a numerical value used in conjunction with the term "about," it is specifically contemplated that the term about can be omitted.

Unless defined otherwise, all technical and scientific terms used herein have the same meaning as commonly understood by those of ordinary skill in the art to which the invention belongs. For the purposes of the present invention, the following terms are defined below.

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used herein, the terms "function" and "functional", and the like, refer to a biological, enzymatic, or therapeutic function.

An "increased" or "enhanced" amount is typically a "statistically significant" amount, and may include an increase that is 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, *e.g.,* 2.1, 2.2, 2.3, 2.4, *etc*.) an amount or level described herein.

A "decreased" or "reduced" or "lesser" amount is typically a "statistically significant" amount, and may include a decrease that is about 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, *etc*.) an amount or level described herein.

The term "multipotent cells" as used herein refers to cultured or uncultured cells that maintain the capacity to differentiate into two or more different specialized cell types. The cells may be live cultured cells or immortalized cells. Alternatively, the cells may be primary cells. In yet another aspect, the cells may be sterilized or preserved. Multipotent cells include stem cells and progenitor cells. Examples of multipotent cells include, but are not limited to, mesenchymal stem cells, embryonic stem cells, induced pluripotent stem cells, neural stem cells, endothelial progenitor cells, adipose-derived stem cells, hematopoietic stem cells, placental stem cells, and umbilical cord stem cells. It is understood that following sterilization or preservation according to the methods described herein, a multipotent cell may lose its capacity to grow or differentiate.

The term "microvascular tissue" refers to tissue that is composed of arterioles, capillaries, venules and associated cellular and extracellular matrix components isolated from nearly any tissue or organ in the body.

The term "processed microvascular tissue" as used herein refers to microvascular tissue that is isolated from the adjacent tissue that it nourishes and then dried using, for example, freeze-drying or spray drying techniques. The processed microvascular tissue provided herein is minimally processed, uncultured microvascular tissue that includes a mixture of stem and/or progenitor cells produced from the dissociation (e.g., by enzymatic digestion) of a microvascular tissue (e.g., adipose, tendon, or muscle tissue). Processed microvascular tissue can include additional molecules (e.g., whole or fragmented extracellular matrix molecules, growth factors, or cell surface molecules).

Processed microvascular tissue and multipotent cell compositions have a variety of biological properties, including anti-inflammatory activity, vascularization activity, mitogenic activity, and soft and hard tissue healing (e.g., repair or regeneration) activity.

The term "obtained from" refers to where a sample such as, for example, a cell or tissue, is derived from, for instance, a particular source, such as a desired organism or a specific tissue within a desired organism. Popular sources for multipotent cells and microvascular tissue include adipose tissue, bone marrow, blood, bone, muscle, amnion, placenta, skin, intestine, and lung.

As used herein, the term "isolated", e.g., with respect to microvascular tissue or multipotent cells, means removed from its natural environment. For example, microvascular tissue is isolated if it is separated from some or all of the coexisting materials in its natural environment. The extent of isolation should increase the concentration of microvascular elements by at least 50% over native tissue. Likewise, isolated multipotent cells should be enriched at least two-fold over their concentration in native tissue.

Cells may be deemed nonviable if they are part of a composition in which <50% of the cells exclude trypan blue and yet at least 1% of the original cells are still visible as cells under phase contrast light microscopy.

The term "bioactivity" refers to the ability of a composition to exert beneficial effects on living matter. As used herein the term "enhanced bioactivity" refers to reduced immune response, anti-inflammatory activity, vascularization activity, wound healing, disease associated pain relief, or a combination thereof. Bioactivity may be measured by cell migration or proliferation assays, expression of certain cellular proteins, reported decrease in pain, medical imaging, increased numbers of capillaries, and/or improved wound healing.

As used herein, the term "anti-inflammatory" refers to the property of a substance that reduces inflammation or swelling.

As used herein, the term "vascularization" refers to the property of a substance to promote angiogenesis, the physiological process through which new blood vessels form from pre-existing vessels or vasculogenesis, the process of blood vessel formation *de novo.* It is a normal and vital process in growth and development as well as in wound healing.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent or emulsifier which has been or will be approved by the United States Food and Drug Administration as being acceptable for use in humans or domestic animals.

A "pharmaceutical composition" refers to a formulation of a composition of the invention and a medium generally accepted in the art for the delivery of a therapeutic agent to mammals, *e.g*., humans. Such a medium includes any pharmaceutically acceptable carriers, diluents or excipients therefore.

As used herein, unless the context makes clear otherwise, "medical treatment," and similar words such as "treated," "treating" etc., indicates an approach for obtaining beneficial or desired results, including clinical results. Treatment can involve optionally either the reduction or amelioration of symptoms of an injury, disease or condition, or the delaying of the progression of the injury, disease or condition. Administration of a composition described herein may, in some embodiments, treat one or more of such symptoms.

As used herein, unless the context makes clear otherwise, "prevention," and similar words such as "prevented," "preventing" *etc.,* indicates an approach for preventing, inhibiting or reducing the likelihood of the onset or recurrence of an injury, disease or condition. It also refers to preventing, inhibiting or reducing the likelihood of the occurrence or recurrence of one or more symptoms of an injury, disease or condition, or optionally an approach for delaying the onset or recurrence of an injury, disease or condition or delaying the occurrence or recurrence of one or more symptoms of an injury disease or condition. As used herein, "prevention" and similar words also includes reducing the intensity, effect, symptoms and/or burden of an injury, disease or condition.

As used herein, an "effective amount" or a "therapeutically effective amount" of a composition is that amount sufficient to affect a desired biological effect, such as, e.g., beneficial clinical results.

The terms "autologous transfer," "autologous transplantation," and the like refer to treatments wherein the tissue donor is also the recipient of the composition produced from the tissue.

The terms "allogeneic transfer," "allogeneic transplantation," and the like refer to treatments wherein the tissue donor is of the same species as the recipient of the composition produced from the tissue, but is not the same individual.

The terms "xenogeneic transfer," "xenogeneic transplantation," and the like refer to treatments wherein the tissue donor is of a different species than the recipient of the composition produced from the tissue.

As used herein, the term "process treatment" or "exposure to treatments" refers to conditions that would be expected to be detrimental to cells or proteins such as radiation, heat shock, extended storage at room temperature or above, long-term cold storage, ultrasonic agitation, fluid shear forces, exposure to hypoxia, reactive oxygen species, electromagnetic fields, and combinations thereof.

As used herein, the term "minimum threshold" or "minimum threshold dose" refers to the quantity to be administered at one time. Herein, it may refer to a given quantity of exposure to a particular treatment. For instance, minimum threshold may refer to a given quantity of radiation as measured in kilogray units. It is understood that the term will have the appropriate units based on the particular treatment. For instance, units of temperature, time, pH, force, or combinations thereof depending on the treatment exposure.

As used herein, the term "kilogray, is a derived unit of ionizing radiation dose in the International System of Units. A gray or "Gy" is defined as the absorption of one joule of radiation energy per one kilogram of matter. It is used as a measure of absorbed dose, specific energy, and kerma (acronym for kinetic energy released per unit mass).

As used herein, the term "maximum limit" is the dose or exposure that leads to loss of integrity of about 90 or about 99% of cells such that the cells can no longer be visualized as cells and counted by phase contrast light microscopy. Integrity refers to cell integrity.

As used herein, "soft tissue" refers generally to extra skeletal structures found throughout the body and includes but is not limited to cartilage tissue, meniscal tissue, ligament tissue, tendon tissue, intervertebral disc tissue, periodontal tissue, skin tissue, vascular tissue, muscle tissue, fascia tissue, periosteal tissue, ocular tissue, pericardial tissue, lung tissue, synovial tissue, nerve tissue, brain tissue, kidney tissue, bone marrow, urogenital tissue, intestinal tissue, liver tissue, pancreas tissue, spleen tissue, adipose tissue, and combinations thereof. Soft tissue injuries include damage or injury to any soft tissue, such as, *e*.*g*., muscles, ligaments, tendons, skin, fibrous tissue, fat, synovial membranes, nerves, blood vessels, and fascia, which may occur throughout the body.

### Multipotent cells

The multipotent cell compositions provided herein comprise, in several embodiments, minimally processed, uncultured cells or uncultured microvascular tissue (or components thereof) that may include a mixture of stem and/or progenitor cells produced from the dissociation (*e*.*g*., by enzymatic digestion) of a microvascular tissue (*e*.*g*., adipose, bone marrow, or muscle tissue). Processed multipotent cell compositions can include additional molecules (*e*.*g*., whole or fragmented extracellular matrix molecules or growth factors). In addition, processed multipotent cell compositions may, comprise fragments or membranes of multipotent cells. The multipotent cells may be purified or cultured. They may be partially differentiated cells, primary cells or cell lines.

Embodiments of the present invention comprise isolated multipotent cells that have been exposed to treatments that result in enhanced bioactivity. In some embodiments, the isolated multipotent cells have been exposed to treatments that decrease or eliminate the ability of the cells to proliferate or differentiate. The treatments include but are not limited to exposure to radiation, storage at room temperature or above, heat shock, hypoxia, exposure to electromagnetic fields, ultrasonic agitation, fluid shear force, pH shock, osmotic shock, long-term cold storage, and exposure to reactive oxygen species. The treatments typically reduce viability of cells or potency of proteins but surprisingly enhance bioactivity of the cell preparations. The treatments often kill the cells but may enhance their ability to release microvesicles, growth factors, and the like at accelerated rates or may simply destroy inhibitory elements. The enhanced bioactivity includes the ability to promote vascularization, wound healing, cell proliferation, cell migration, and reduce inflammation or a combination thereof.

The present invention relates to methods of making multipotent cells with enhanced bioactivity comprising isolating multipotent cells and exposing the cells to treatments such as radiation, heat, or shear forces that result in enhanced bioactivity.

The methods of the present invention may be used to prepare a composition comprising multipotent cells alone or in combination with one or more additional cell types and/or other components.

In particular embodiments, the additional cell type is a stromal, epithelial, or blood-derived cell, including, but not limited to, fibroblasts, keratinocytes including follicular outer root sheath cells, endothelial cells, pericytes, red blood cells, monocytes, lymphocytes including plasma cells, neutrophils, thrombocytes, mast cells, adipocytes, muscle cells, smooth muscle cells, hepatocytes, nerve and neuroglia cells, osteocytes, and osteoblasts.

In particular embodiments, the additional tissue component is a component of the extracellular matrix. The extracellular matrix comprises diverse constituents such as proteins, glycoproteins, proteoglycans, complex carbohydrates, and other molecules. The extracellular matrix may comprise any of a number of different proteins, including various collagens, elastin, fibronectin, laminin, vitronectin, thrombospondin, tenascin (cytoactin), entactin (nidogen), osteonectin (SPARC), anchorin CII, chondronectin, link protein, osteocalcin, bone sialoprotein, osteopontin, epinectin, hyaluronectin, amyloid P component, fibrillin, merosin, s-laminin, undulin, epilligrin, kalinin, fibrin, fibrinogen, and HSP.

In related embodiments, the additional tissue component comprises a growth factor, a vascularization agent, an anti-inflammatory agent, a cytokine, or a differentiation agent. For example, a growth factor or vascularization agent may be selected from basic fibroblast growth factor, other fibroblast growth factors, bone morphogenetic proteins, hepatocyte growth factor, keratinocyte growth factor, granulocyte macrophage colony stimulating factor, platelet-derived growth factor, transforming growth factor beta1 and/or beta3, and vascular endothelial cell growth factor. Additional growth factors and classes or families of growth factors that may be used include any of those known in the art.

### Microvascular Tissue

Processed microvascular tissue described herein may be produced by dissociating a microvascular tissue. In some embodiments, the microvascular tissue is enzymatically digested using one or more enzymes. Suitable enzymes include those that contribute to tissue dissociation, such as collagenases and neutral proteases such as thermolysin or BP protease. The enzymatic digestion process can be adjusted to increase or decrease tissue dissociation. For example, if more complete dissociation is desired, more than one enzyme can be included or digestion time can be increased. While cell viability need not be maintained, in some embodiments it is generally desired that cellular membranes remain generally intact to preserve membranes containing attachment and signaling molecules even if some cell lysis occurs during enzymatic digestion. Thus, the use of enzymes such as lipases may not be useful in such a process, according to one embodiment of the present invention. In such a scenario, a collagenase or neutral protease may be utilized.

Alternatively, the microvascular tissue can be dissociated without the use of enzymes. Rather, microvascular tissue can be dissociated using physical or chemical means, including the use of chelators, ultrasonic agitation, or mechanical cell dissociation. Mechanical cell dissociation may include gentle pipetting, manual scraping, nylon mesh, mechanical disruption, and the like.

The procurement of donor microvascular tissue and subsequent treatment can include steps for preventing microbial (*e*.*g*., bacterial, fungal, or viral) contamination. For example, donors can be screened for a predetermined list of microbial organisms (*e*.*g*., HIV, HPV, EBV, TB, *etc*.) prior to processing. Screening can be done using known techniques, such as detecting the presence of a microbial nucleic acid using polymerase chain reaction, or by detecting the presence of a molecule associated with a particular microbe by ELISA. Microbial contaminated microvascular tissue can be excluded from use, according to some embodiments of the present invention. In addition, microvascular tissue can be produced using aseptic or sterile techniques, or terminally sterilized.

Following tissue dissociation, microvascular tissue can be further treated to remove undesired cells or molecules, such as red blood cells, lipids, or adipocytes. Additional treatment will depend upon the source of microvascular tissue and its intended use. For example, if the microvascular tissue source is adipose tissue, the dissociated microvascular tissue can be centrifuged at relatively low force to separate lipids, adipocytes, and some pre-adipocytes from the rest of the microvascular tissue. In other embodiments, known muscle cell isolation protocols, such as the use of density gradient centrifugation, may be used to further treat muscle tissue following enzymatic digestion to remove muscle cells and enrich for microvascular associated cells and tissue.

Once the microvascular tissue is prepared, it may be preserved by drying using, for example, a freeze-drying or spray-drying technique to produce processed microvascular tissue. Any appropriate excipient can be used when preserving microvascular tissue, including sugars (*e*.*g*., trehalose, mannitol, sucrose), polyalcohols (*e*.*g*., polyethylene glycol), aldehydes, proteins (*e*.*g*., albumin), amino acids (*e*.*g*., glycine), surfactants (*e.g.,* Tween 20), DMSO, and/or permanganates.

Freeze drying typically involves four steps: pretreatment, freezing, primary drying, and secondary drying. Pretreatment can include concentration adjustment or the addition of one or more excipients. Following pretreatment, the microvascular tissue is frozen. The freezing step is typically done in a carefully controlled manner (*e*.*g*., at a rate of cooling of between about -0.5 °C per minute to about -50 °C per minute) to preserve cell structure, however cell viability need not be preserved. In some embodiments, microvascular tissue is frozen at a rate of cooling of about -10°C per minute. The rate of cooling can be adjusted based on the particular microvascular tissue and excipients used. The microvascular tissue can be frozen using any appropriate means, including using mechanical refrigeration and/or exposing a container containing the microvascular tissue to dry ice or liquid nitrogen until it reaches a temperature suitable for freeze-drying.

Additionally, because viability is not required for suitability of the processed microvascular tissue for use in tissue repair, the preservation process and storage need not be adjusted to maintain viability. The percentage of viable cells in the provided microvascular tissue before processing can be up to 100%. After processing, it may be less than 50%, e.g., less than 40%, less than 30%, less than 20%, less than 10%, less than 5%, or less than 1%. In some embodiments, the provided processed microvascular tissue contains no measurable viable cells after processing or cryopreservation.

In some embodiments of the present invention, microvascular tissue is prepared as described in Example 1.

### Production treatment methods

Sterilization of tissues typically causes great damage to proteins and cells. The proteins are often denatured, DNA is cleaved and/or cross-linked, and cells are killed.

To minimize damage, such products are processed very carefully to reduce bioburden. They may then be sterilized with radiation exposures as low as 14-15 kGy. Surprisingly, the multipotent cell compositions described herein have improved potency as measured by bioactivity when exposed to 40 kGy radiation.
Radiation may be achieved by exposure to a range of wavelengths from electron-beam ("E-beam") to infrared as illustrated in the Examples below.

The surprising phenomenon of enhanced bioactivity with processing that typically reduces potency of cells or proteins may be seen under a variety of circumstances. Compositions of the present invention show enhanced bioactivity when stored for prolonged periods of time or exposed to elevated temperatures. Exposing the compositions to hypoxic conditions, reactive oxygen species, electromagnetic fields, ultrasonic agitation, fluid shear force, non-physiologic pH or tonicity also leads to improved potency. The composition may comprise multipotent cells, processed microvascular tissue or a combination thereof. Multipotent cells include but are not limited to purified, cultured cells, non-cultured cells, and partially differentiated cells from primary tissue or cell lines. The multipotent cells may be sterilized and dried.

Examples of storage conditions include long-term storage at room temperature. Long term may be about 6 months, less than 1 year, about 1 year, about 2 years, or longer than 3 years. Room temperature is about 25°C. Storage conditions may be storage in cold temperatures such as 0°C, -20°C, or -80°C, or at elevated temperatures up to 46°C.

Examples of hypoxic conditions include for example below 3% oxygen. Hypoxic conditions may be generated with a CO₂ incubator. Alternatively, hypoxic conditions may be created in a "tri-gas" incubator. However, only two gases are supplied-carbon dioxide (as usual) and nitrogen-to reduce the oxygen levels. Generally oxygen can be lowered below 1%.

Examples of exposure to reactive oxygen species, which are chemically reactive molecules containing oxygen, include peroxides, superoxide, hydroxyl radical, peracetic acid, and singlet oxygen.

An example of electromagnetic field exposure includes microwave energy.

Sonication is the act of applying sound energy to agitate particles in a sample, for various purposes. Ultrasonic frequencies (>20 kHz) are usually used, leading to the process also being known as ultrasonication. In biological applications, sonication may be sufficient to disrupt or deactivate a biological material. For example, sonication is often used to disrupt cell membranes and release cellular contents. This process is called sonoporation. Sonication is also used to fragment molecules of DNA, in which the DNA subjected to brief periods of sonication is sheared into smaller fragments.

Shear stress is defined as the component of stress coplanar with a material cross section. Shear stress arises from the force vector component parallel to the cross section. Normal stress, on the other hand, arises from the force vector component perpendicular to the material cross section on which it acts. Shear stress is a fundamental determinant of vascular homeostasis, regulating vascular remodeling, cardiac development and atherogenesis. Fluid shear force can be mimicked in laboratory setting by exposure to laminar or turbulent flow. Convenient methods of shearing the product include vigorous mixing or forcing through a small orifice.

Examples of non-physiologic pH include pH less than 7.0 or above 7.8. This includes ranges from about 0 to about 7.0 and from about 7.8 to about 14. This includes ranges within those limits for example from about 0 to about 1, from about 0 to about 2, from about 0 to about 3, from about 0 to about 4, from about 0 to about 5, from about 0 to about 6, from about 0 from about 7, from about 7.8 to about 8, from about 7.8 to about 9, from about 7.8 to about 10, from about 7.8 to about 11, from about 7.8 to about 12, from about 7.8 to about 13, and from about 7.8 to about 14.

### Bioactivity

In particular embodiments, a composition of the present invention has one or more biological activities. For example, in certain embodiments, a composition has anti-inflammatory, vascularization activity, or a combination thereof. A composition may elicit migration or proliferation of endothelial cells. In certain related embodiments, a composition promotes blood vessel formation or tissue healing. Combinations of these effects are achieved in several embodiments.

In certain embodiments, a composition of the present invention has anti-inflammatory activity. In particular embodiments, an injured or diseased tissue (*e*.*g*., an injured or diseased tissue undergoing an inflammatory response) exposed to or contacted with a composition of the present invention exhibits reduced inflammation as compared to when the injured or diseased tissue is similarly treated but not exposed to or contacted with the composition of the present invention. In certain embodiments, the amount of inflammation in the tissue exposed to or contacted with the composition of the present invention is reduced by at least about 10%, at least about 20%, at least about 30%, at least about 40%, at least about 50%, at least about 60%, at least about 70%, at least about 80%, or at least about 90%, as compared to the amount of inflammation when the injured or diseased tissue is not exposed to or contacted with the composition of the present invention. Inflammation may be measured by means available in the art, including, *e*.*g*., the number of lymphocytes observed in the affected tissue when observed histologically.

In particular embodiments, a composition of the present invention has anti-inflammatory activity that may be measured as pain or visually. Visually it may be measured by redness and/or swelling.

In certain embodiments, a composition of the present invention has vascularization activity. In particular embodiments, an injured or diseased tissue (e.g., an injured or diseased tissue undergoing an inflammatory response) exposed to or contacted with a composition of the present invention exhibits increased angiogenesis as compared to when the injured or diseased tissue is similarly treated but not exposed to or contacted with the composition of the present invention. In certain embodiments, the amount of angiogenesis in the tissue exposed to or contacted with the composition of the present invention is increased by at least about 10%, at least about 20%, at least about 30%, at least about 40%, at least about 50%, at least about 60%, at least about 70%, at least about 80%, at least about 90%, at least about 100%, at least about 150%, at least about 200%, at least about 300%, at least about 400%, or at least about 500%, as compared to the amount of angiogenesis when the injured or diseased tissue is not exposed to or contacted with the composition of the present invention. Angiogenesis may be measured by means available in the art, including, *e*.*g*., the hind limb ischemia model.

In particular embodiments, a composition of the present invention has vascularization activity that may be measured in an *in vivo* or *in vitro* assay. In certain embodiments, the amount of activity measured in an *in vitro* angiogenesis assay in the presence of a composition of the present invention is at least about 10%, at least about 20%, at least about 30%, at least about 40%, at least about 50%, at least about 60%, at least about 70%, at least about 80%, at least 90 about %, at least about 100%, at least about 150%, at least about 200%, at least about 300%, at least about 400%, or at least about 500% greater than the amount of activity measured in the same assay in the absence the composition of the present invention or in the presence of a control composition. In particular embodiments, the *in vivo* assay is a matrigel assay. In particular embodiments, the *in vitro* assay is the endothelial cell migration assay described herein.

In certain embodiments, a composition of the present invention promotes healing of an injured or diseased tissue, *i.e.,* it has tissue healing activity. As used herein, "tissue healing activity" of a composition is the ability of the composition to facilitate improved healing (*e*.*g*., repair or regeneration) of an injured or diseased tissue (e.g., a hard or soft tissue) exposed to the composition as compared to an analogous tissue similarly treated but without exposure to the composition. Improved healing is measured using any appropriate means, such as time to complete healing, amount of new tissue generated, strength of the resulting healed tissue, or functionality of the resulting healed tissue.

Sterilized or virus-inactivated allogeneic and xenogeneic multipotent cell and processed microvascular tissue compositions have not previously been used to facilitate repair of soft tissues such as ligaments and tendons, because of the difficulty of producing new soft tissue with autologous stem cells, the perception that allogeneic and xenogeneic stem cells will be rejected, and the prior belief that sterilized or virus-inactivated cells will have reduced viability and, thus, reduced biological or therapeutic activity. However, the process and compositions described herein do not necessarily rely on autologous stem cells or cell viability. Rather, the provided process is used to produce a composition containing stem cells, or progenitor cells, or mixtures of such cells even with other cell types and molecules associated with such cells (*e*.*g*., cytokines, growth factors, chemotactic molecules, and the like) that have been exposed to a post-processing treatment to enhance bioactivity. Such treatments include but are not limited to irradiation, room temperature storage, cold temperature, hypoxic conditions, reactive oxygen species, electromagnetic fields, ultrasonic agitation, fluid shear force, non-physiologic pH or tonicity. In some embodiments, the processing treatments are performed before drying or sterilizing. In other embodiments, the composition contains a mixture of viable and nonviable cells. In certain embodiments the composition contains differentiated or engineered therapeutic cells that have been exposed to one or more of the disclosed processes to enhance bioactivity.

In particular embodiments, less than about 50%, less than about 40%, less than about 30%, less than about 20%, less than about 10%, less than about 5%, less than about 3%, or less than about 1% of the cells present in a composition of the present invention are viable. In several embodiments, substantially all of the cells are non-viable. As used herein, the term "viable" shall be given its ordinary meaning and shall also refer to a cell that is capable of proliferating when cultured under appropriate conditions, e.g., conditions under which the same cell or type of cell would be expected to proliferate, e.g., if not processed as described herein. In other embodiments, less than about 2% or less than about 1% of the cells present in said composition are viable. In particular embodiments, none or substantially none of the cells present in the composition are viable.

Accordingly, the term "non-viable" means that the cell is not capable of proliferating when cultured under appropriate conditions, e.g., conditions under which the same cell would be expected to proliferate, e.g., if not processed as described herein. Examples 2 through 5 show exemplary the bioactivity of processed microvascular tissue of certain aspects of the present invention. Post-production processing treatments that were tested include irradiation (Example 2), storage time at room temperature (Example 3), storage temperature (Example 4), and hypoxia (Example 5).

### Methods of Use

In certain embodiments, compositions of the present invention are for use in the treatment of disease conditions.

Tissue injuries, disease and conditions that may be treated or prevented according to the present invention include, but are not limited to, injuries to vascular, skin, or musculoskeletal tissue. Musculoskeletal conditions include, for example, arthritis, tendinopathies, torn ligaments, bone fractures, osteomyelitis, cleft palate, myofascial pain syndrome, joint hypermobility/Ehlers-Danlos syndrome, Complex Regional Pain Syndrome (CRPS), and ailing dental implants. Soft tissue conditions include, for example, conditions of skin (e.g., lichen sclerosis, scar revision, scar tissue such as in Peyronie's disease, or the treatment of traumatic wounds, severe burns, skin ulcers [*e*.*g*., decubitus (pressure) ulcers, venous ulcers, and diabetic ulcers], surgical wounds such as those associated with the excision of skin cancers), and eczema; vascular condition (*e*.*g*., vascular disease such as peripheral arterial disease and venous disease; vascular injury; improper vascular development; avascular necrosis); conditions affecting vocal cords or periodontal tissues; cosmetic conditions (*e*.*g*., those involving repair, augmentation, or beautification including bulking agents, fat grafting, hair growth, breast reconstruction and healing following dermal abrasion procedures); muscle injuries; muscle diseases (*e*.*g*., congenital myopathies; myasthenia gravis; inflammatory, neurogenic, and myogenic muscle diseases; and muscular dystrophies such as Duchenne muscular dystrophy, Becker muscular dystrophy, myotonic dystrophy, limb-girdle-muscular dystrophy, facioscapulohumeral muscular dystrophy, congenital muscular dystrophies, oculopharyngeal muscular dystrophy, distal muscular dystrophy, and Emery-Dreifuss muscular dystrophy); conditions such as peripheral neuropathy; and conditions of organs and/or fascia (*e.g.,* the bladder, intestine, pelvic floor) such as interstitial cystitis. One example of a fairly common soft tissue injury is damage to the pelvic floor. This is a potentially serious medical condition that may occur during childbirth or from complications thereof, which can lead to damage to the vesicovaginal fascia, such as a cystocele, which is a herniation of the bladder. Similar medical conditions include rectoceles (a herniation of the rectum), enteroceles (a protrusion of the intestine through the rectovaginal or vesicovaginal pouch), and enterocystoceles (a double hernia in which both the bladder and intestine protrude).

In certain embodiments, compositions of the present invention are for use in the treatment or prevention of a soft tissue injury.

Soft tissue injuries that can benefit from the soft tissue healing activity of the provided enhanced compositions include, without limitation, injuries such as tendon and/or ligament tears and injuries resulting from ischemic events. Common soft tissue injuries may result from sprain, strain, an injury resulting in a contusion, or overuse of a particular soft tissue. Soft tissue injuries include both open and closed soft tissue injuries.

In various embodiments, compositions of the present invention are for use in the treatment or prevention of various diseases, including but not limited to diseases associated with undesirable inflammatory or immune responses. Examples of such disease include rheumatoid arthritis, osteoarthritis, bursitis, and autoimmune diseases and disorders such as Crohn's disease and autoimmune hepatitis. In addition, compositions of the present invention may be used to reduce inflammation, *e*.*g*., at a site of injury, and/or to reduce an immune response, *e*.*g*., an immune response induced by an injury. Similarly, compositions of the present invention may be used to prevent or reduce the likelihood or severity of transplant rejection.

In particular embodiments, compositions of the present invention are for use in the promotion or stimulation of angiogenesis or revascularization, e.g., at a site of injury or tissue damage. In particular embodiments, the injury is associated with or resulted in ischemia, hypoxia, or reperfusion injury to a tissue. Examples of injuries or diseases associated with or resulting in ischemia, hypoxia, or reperfusion injury that may be treated or prevented according to the present invention include stroke, myocardial infarct, acute kidney injury, and blood loss. Additional examples of injuries or tissue damage that may be treated with compositions of the present invention to promote or stimulate angiogenesis or revascularization include transplantation or limb reattachment.

Compositions of the present invention may be for use in treating or alleviating pain associated with a medical condition. Examples of such medical conditions include plantar fasciitis, arthritic joints, tendinopathies, back pain, claudication, myofascial pain syndrome, and temporomandibular joint pain.

Compositions of the present invention may also be for use in the treatment or prevention of peripheral nerve damage, erectile dysfunction, multiple sclerosis, and radiation burns. In addition, they may be used to induce hematopoiesis and/or wound healing. They may also be used to treat diabetic nephropathy, diabetic retinopathy, and macular degeneration.

Compositions described herein, but not claimed, *e*.*g*., when formulated for intravenous administration, may be used to treat or prevent acute myocardial infarct, congestive heart failure, stroke, peripheral vascular disease, pulmonary hypertension, emphysema or chronic obstructive pulmonary disease. Respiratory problems may also be treated with a milled form of the present invention suitable for inhalation.

The compositions of the present invention may be used alone or in combination with one or more other therapeutic agents or procedures to treat or prevent an injury or disease. For example, in certain embodiments, to enrich blood supply to a damaged tissue and/or to promote tissue regeneration, compositions of the present invention may be used in combination with platelet-rich plasma. When used in combination with one or more other therapeutic agents or procedures, the compositions of the present invention may be provided or used prior to, at the same or during an overlapping time period as, or subsequent to, treatment with the other therapeutic agent or procedure.

Further provided are methods of using processed microvascular tissue. Processed microvascular tissue can be applied directly to a tissue in need of repair, or can be applied to tissue surrounding such a tissue in need of repair. In some embodiments, a dried processed microvascular tissue is reconstituted in a suitable carrier (*e*.*g*., water or saline) and directly applied to a tissue in need of repair. Experience has shown that solutions containing anesthetic such as Marcaine are preferred by some patients. For topical use processed microvascular tissue may be applied as a lyophilized cake, as a powder, mixed into a dermatologic cream, as a dried film or as a solution. In some embodiments, reconstituted processed microvascular tissue is applied to a scaffold, such as a collagen matrix, fibrin clot or biocompatible fabric, prior to being applied to a tissue. In other embodiments, a processed microvascular tissue is used to coat a material, such as a flexible biocompatible scaffold (e.g., woven or nonwoven fabric sheets or thread), biocompatible microbeads or particles, or an implantable medical device. Spray dried processed microvascular tissue is particularly suited to coating a material comprising microbeads or particles without requiring reconstitution prior to coating, as coating can be done during the spray drying process.

Processed microvascular tissue can be combined with any suitable device or material prior to implant into a patient. Processed microvascular tissue can be combined with an orthopedic implant; a porous, flexible implantable scaffold; a surgical implant; pure water; saline; anesthetic solution; a porous coated implant; polymer solution; solvents such as DMSO, N-methylpyrrolidone (NMP), and alcohols; hydrogel; hyaluronic acid or other glycosaminoglycans or proteoglycans; collagen; fibrin; thrombin; blood clot; platelets; platelet rich plasma; demineralized bone matrix; autologous cells; and/or bone or bone components.

Processed or cryopreserved microvascular tissue can be packaged alone for example, in a vial, or in combination with other products, such as those listed as being suitable for combination with processed or cryopreserved microvascular tissue. When packaged with another material, the processed or cryopreserved microvascular tissue can be separately packaged, or premixed or associated with the other material. In some embodiments, processed microvascular tissue is packaged as a coating on a biocompatible material.

It has been previously shown in animals that nonviable multipotent cells improved healing of tendons (US Patent No. 9,044,430). Clinical use of the enhanced compositions of the present invention has shown rapid healing of a variety of tendinopathies and surprising pain relief (Example 7). The pain relief has also been observed in plantar fasciitis (Example 9), a chronic painful condition of the foot with no clear etiology and no other effective treatment except extended unloading of the foot. In arthritic joints, injection of processed microvascular tissue of the present invention has provided profound, durable pain relief without apparent healing of the cartilage as imaged by MRI or X-ray (Example 6).

Clinical use has shown unexpected benefits in the treatment of chronic wounds (Example 8), scars, tendinopathies, and osteoarthritis. In addition to the healing noted in such patients there is a marked reduction in pain. This pain reduction has also been observed in patients treated for temporomandibular joint pain (Example 10).

Some embodiments of the present invention comprise compositions with no viable cells yet show benefits in a number of clinical uses. Chronic wounds that have not healed when treated with conventional therapies such as hyperbaric chambers, colloidal dressings, tissue grafting, and unloading heal in as little as one week following application of enhanced, processed microvascular tissue of the present invention. A lyophilized cake of processed microvascular tissue of the present invention can be applied directly to the wound, powdered and spread over the surface of larger wounds, or hydrated with water or other appropriate fluids and injected subcutaneously in and around the wound. The wounds are then typically covered with traditional dressings.

The methods of the present invention may be practiced by administering the composition in one, two or more doses. For example, in certain embodiments, a composition is administered as a single dose, multiple doses or in repeated doses over a period of time.

### EXAMPLES

### Example 1: Preparation of Processed Microvascular Tissue

Fat tissue was obtained from an organ donor. Subcutaneous fat is taken from abdomen, thighs and buttocks. Five (5) to ten (10) pounds of fat were harvested into an appropriate media, such as ZTM^{™} transport medium (INCELL).

The tissue was harvested and initially processed within a day of death by washing with PBS and then suspending in CIzyme HA (Vitacyte) plus neutral protease enzymes in ZSolM^{™} (INCELL) at 37 °C for 40 minutes.

After digestion, the samples were rinsed in an appropriate buffer such as PBS. In this experiment, ZSolF^{™} was used and the samples were then centrifuged, decanted, and then washed two more times in ZSolF^{™}. The cells were resuspended in 1:1 mix of M3D^{™} in EZ-CPZ^{™} (INCELL) cryoprotectant solution at 1.5 × 10⁶ cells/ml and lyophilized in 1 mL aliquots.

### Example 2: Effect of Irradiation (only the composition sterilized with 40 kGy radiation falls within the claims)

Compositions prepared as described in Example 1 were sterilized by radiation exposure and then tested for bioactivity using human umbilical vein endothelial cells (HUVEC) in a migration assay. The HUVEC were first passage cells cultured until log growth phase and then labeled fluorescently with CM-DiI (Invitrogen). Transwell plates (ThermoFisher) were prepared with cell culture media (50:50 mix of M3D^{™} and EZ-CPZ^{™} INCELL) alone (negative control), supplemented with epithelial cell growth factor (EGF - positive control), or with rehydrated compositions of microvascular tissue that had been treated with various types and doses of radiation. The labeled HUVEC cells were placed in the upper chamber of the Transwell plates and cultured for 48 hours at 37°C in a cell culture incubator. The PET membranes separating the upper and lower chambers had 3 um pores. After 48 hours the upper chambers were removed and the fluorescence intensity in the lower chambers was measured.

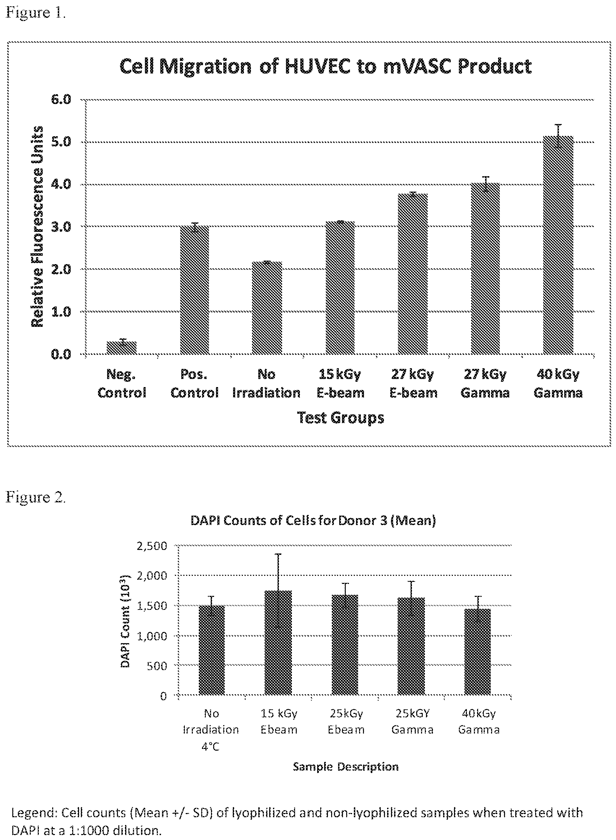

The experimental results are graphed in Figure 1. The bioactivity increased with increasing dose of radiation, irrespective of wavelength, and even exceeded the positive control. Aliquots of the treated compositions were stained with DAPI (nuclear stain) and counted on a hemocytometer with a fluorescence microscope. The cell counts (Figure 2) indicate that the irradiation did not have a significant effect on the number of cells in the treated compositions.

### Example 3: Effect of Storage Time (not according to the claims)

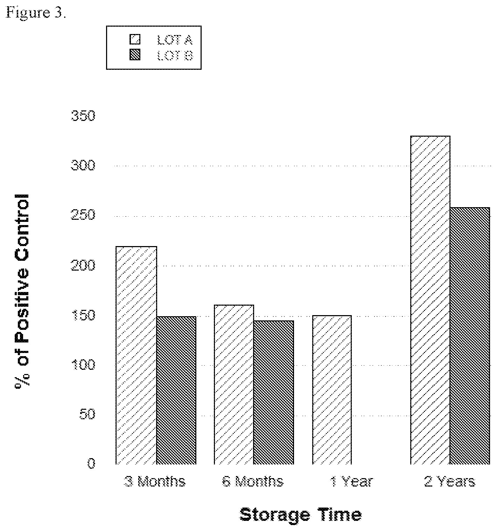

Compositions prepared as described in Example 1 and then sterilized with 27 kGy of gamma irradiation were stored at room temperature and then assayed with the HUVEC migration assay at various times. As shown in Figure 3, the samples stored for two years surprisingly showed increased bioactivity compared to the earlier time points.

### Example 4: Effect of Storage Temperature (not according to the claims)

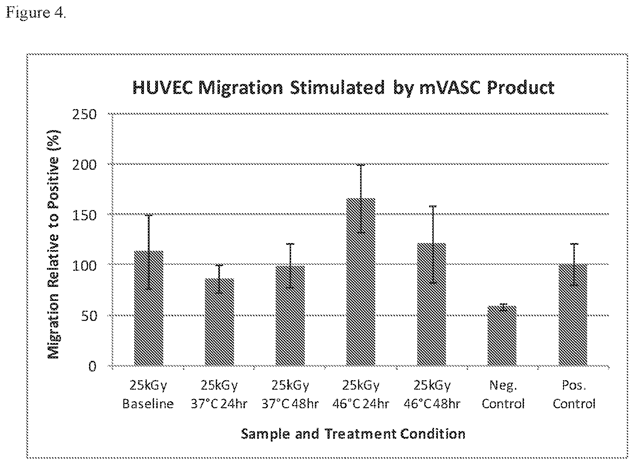

Compositions prepared as in Example 1 and sterilized with 25 kGy of gamma radiation were exposed to elevated temperatures to simulate possible shipping situations. The baseline samples were held at room temperature. Unexpectedly, the overheated samples showed the highest bioactivity as shown in Figure 4.

In a separate, though related experiment, samples that had not been irradiated were stored for six months at room temperature or below. As shown in Table 1 the bioactivity was reduced at lower storage temperatures.

**Table 1: Bioactivity of non-irradiated samples stored 6 months**

| **Storage Temperature** | **Relative Migration** |
|---|---|
| RT | 40% |
| 4 °C | 55% |
| -20 °C | 35% |
| -80 °C | 5% |

### Example 5: Effect of Oxygen (not according to the claims)

During the processing in Example 1, the cellular composition is subjected to low oxygen levels. The expression of certain cellular proteins will be enhanced or inhibited as a result, this affecting the functionality of the composition. Hypoxia is associated with inducing expression of Vascular Endothelial Growth factor (VEGF), an important factor in promoting angiogenesis and vascularization. Thus, exposure to hypoxia can be used as a means by which expression of VEGF may be enhanced in the administered composition.

### Example 6: Treatment of Arthritis (not according to the claims)

A 57-year-old male farmer was suffering from chronic pain due to osteoarthritis which he rated at 8-9 on a 10 point pain scale. The pain was limiting his work and recreational activities. A vial of processed microvascular tissue prepared as in Example 2 was hydrated with 3 ml of water and the solution was injected into the affected knee. At the five week follow-up visit the pain had subsided to zero. The subject stated that the pain was gone within a week of the injection and he had resumed full activities on the farm and had gone on a bike ride for the first time in years.

The subject had magnetic resonance images taken before the injection and again 4 months later. The radiologist's reports on the images are summarized as follows:

PRE-TREATMENT: Pertinent findings include 1) Effusion: Small to moderate supra-patellar effusion; 2) Subcutaneous soft tissues: Moderate subcutaneous fat edema and fluid medial to iliotibial band and extending to the popliteal fat pad; 3) Lateral collateral ligament: Proximal tendinopathy and/or partial tear; 4) Patella-femoral joint: Grade I CP; 5) Medial joint compartment: Near full thickness cartilage loss involving more than 70% of articular surface of both the distal lateral condyle of the femur and lateral tibial plateau, small focal ulcerations of the residual cartilage, and very minimal adjacent reactive (sclerotic) bone changes and no subchondral geodes; 6) Bone marrow: Moderate size bone marrow contusion in the medial-posterior lateral condyle and marrow contusion of the patella; and 7) Cortex: No fracture.

FOUR MONTHS POST-TREATMENT Pertinent Findings include 1) Effusion: Small - resolving; 2) Subcutaneous soft tissues: Significant resolution of the subcutaneous fat edema with a minimal residual amount; 3) Lateral collateral ligament: Resolving acute tendinopathy; 4) Patella-femoral joint: Grade I CP, no change; 5) Medial joint compartment: No change; 6) Bone marrow: No change in marrow contusions; and 7) Cortex: No fracture.

### Example 7: Treatment of Tendinopathy (not according to the claims)

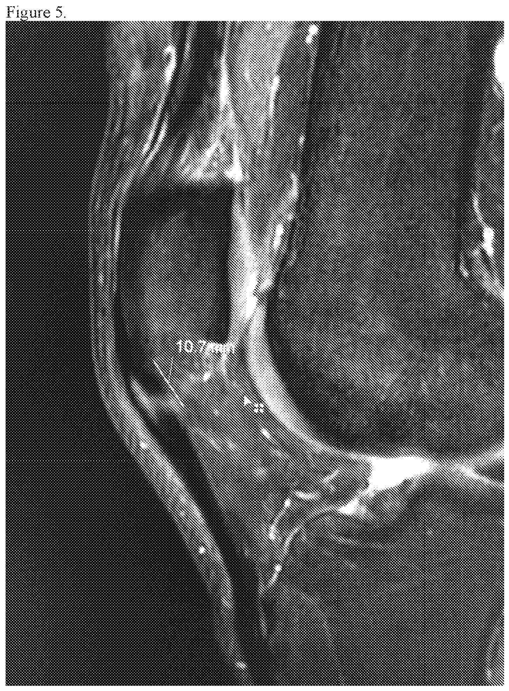

Patient was a 40-year-old male athlete with recent severe and progressive pain for the past 3-4 months to the inferior region of the right kneecap, which was elicited by performing squats without weights. He was unable to tolerate any pressure on the inferior kneecap by palpation. On a pain scale of 0 to 10, 10 being the worst pain, he stated the pain is around 8 - 9 when performing a squat without weights and about a nine when putting any pressure on this region. He commented that the pain might have been caused by performing a very deep squat and then jerking to a standing position with his knees slightly hyper flexed. He does not experience much if any pain while walking or resting. Figure 5 is the pretreatment MRI of the knee showing a tendon tear.

One (1) vial of processed microvascular tissue prepared as in Example 2 was injected into patellar tendon, and one (1) vial of processed microvascular tissue prepared as in Example 2 was injected into the gluteus minimus.

Five weeks after the injection, the patient had no pain to the infrapatellar region with firm palpation. He was also able to perform deep squats without weights without any pain.

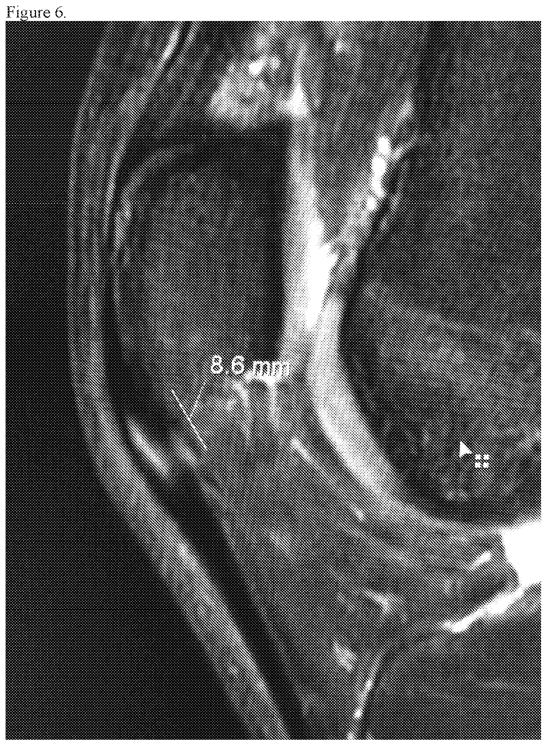

Six weeks after the injection he began performing squats with weights about 70% less than his maximum weight without any pain. Figure 6 is a post-treatment MRI showing healing of the affected tendon.

### Example 8: Treatment of Chronic Wound (not according to the claims)

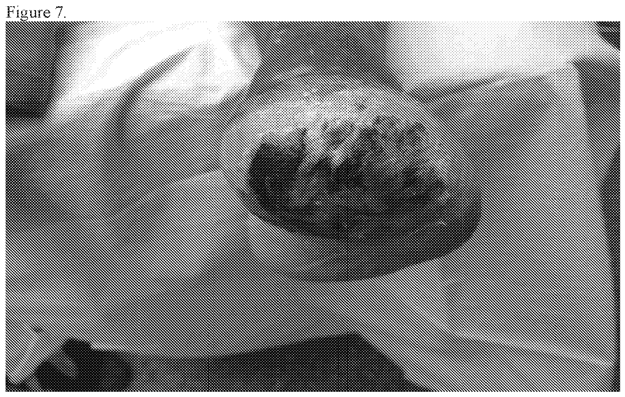

A 48-year-old female with diabetes and renal failure was treated for a year by a wound clinic for a large diabetic foot ulcer. The wound was debrided down to tendon in some regions exposing a wound 5 cm x 3.5 cm x 0.4 cm (as shown in Figure 7) and a vial of processed microvascular tissue prepared as in Example 2 was applied topically with an Adaptic dressing by crushing the lyophilized cake and sprinkling across the surface of the wound.

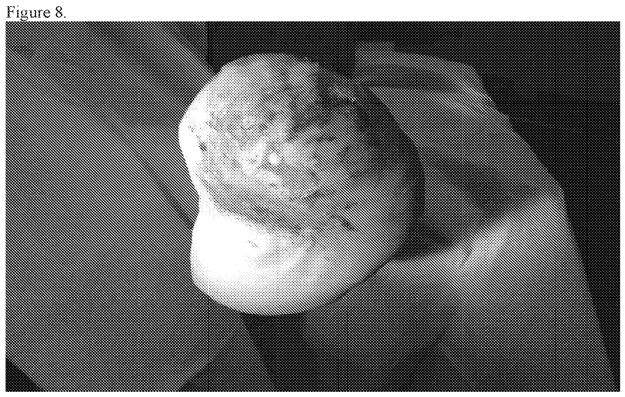

One week later the subject returned for follow-up and showed extensive healing of the ulcer as shown in Figure 8. The wound went on to full closure during the subsequent weeks.

### Example 9: Treatment of Plantar Fasciitis (not according to the claims)

Subject was a 47-year-old female. She had prior surgery to her left foot for a tarsal tunnel release and medial plantar fascia release. Fifteen (15) weeks post-op the patient was complaining of severe pain (rates pain 8 out of 10). She had failed standard therapy, which included: cortisone injection, gastroc recession as well as a medial plantar fascial release, and outpatient physical therapy.

Treatment: After prepping the treatment area with betadine, an injection with 1cc of processed microvascular tissue (with WFI) prepared as in Example 2 was made to the left heel through a plantar approach using a 25g needle, processed microvascular tissue was injected down to the level of the calcaneal tuberosity and then back out to ~5mm. The patient tolerated injection well. The patient was also put into a CAM boot.

One (1) Week Follow-up post processed microvascular tissue treatment: Patient stated that since injection to left heel, she no longer had pain in the heel and a zero out of 10-pain level at the plantar fascia origin.

### Example 10: Treatment of Temporomandibular Joint (TMJ) Pain (not according to the claims)

Forty-five (45) year-old female had constant jaw pain in both the left and right condyles. She rated the pain equally at 8-9 out of 10. A 1cc injection of processed microvascular tissue prepared as in Example 2 (reconstituted with Marcaine 0.25% without Epinepherine) was made inferior, superior and lateral to the right condyle within the joint space using a 25g needle. The contralateral side (left condyle) was then treated exactly the same as the right using Celestone (betamethasone).

One (1) week after treatment the patient rated the pain score of 0 for right side (processed microvascular tissue treated side). Patient remarked that right side was pain free the day after injection. Her left side was 4-5 on pain scale of 10.

Six weeks after injection of steroid in the left condyle the patient's pain level was at 6-7 out of 10. The left condyle was treated with processed microvascular tissue.

### Example 11: Preparation of Microvascular Tissue from Adipose Tissue without use of Enzymes (not according to the claims)

Twenty grams of subcutaneous fat harvested from a human cadaveric donor was added to each of two 50 ml centrifuge tubes. Five ml of Hank's basic salt solution was added to centrifuge tube A, mixed, and then the tube was placed in a 37 C ultrasonic water bath and agitated with ultrasonic energy for 35 minutes. A large magnetic stir bar was dropped into centrifuge tube B, the tube was capped, and the tube was shaken vigorously for 5 minutes so that the stir bar bounced from end to end crushing the fat tissue and lysing the adipocytes. The tubes were filled to 50 ml with Hanks BSS and then centrifuged for 7 min at 300 g. The oil and buffer were poured off and the remaining tissue pellet was poured into petri dishes forming a thin slurry in the bottoms of the petri dishes. The Petri dishes were then frozen and lyophilized producing thin flexible films of microvascular tissue. The films were sterilized using 27 kGy gamma radiation.

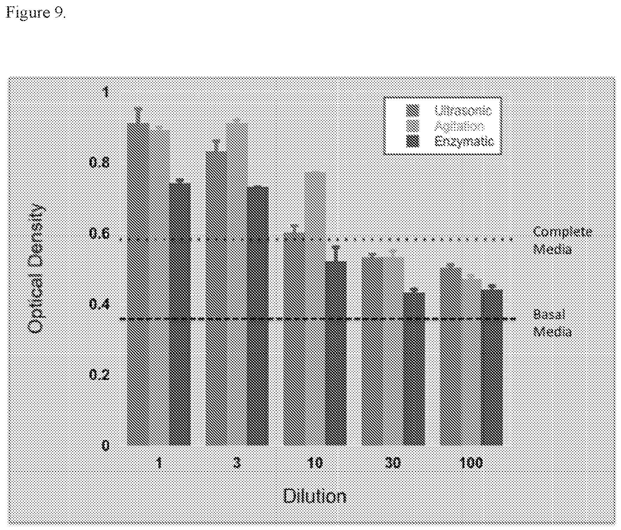

The activity of the films of microvascular tissue was assayed using a cell proliferation assay and compared to microvascular tissue that was prepared enzymatically as described above. The films and cake were rehydrated using basal cell media and then serially diluted into basal media containing human umbilical vein endothelial cells (HUVEC). After two days the cells were labeled with a fluorescent dye and the relative numbers determined by fluorescent intensity in the wells as shown in Figure 9. Both films showed activity as good as or better than the enzymatically digested microvascular tissue.

### Example 12: Treatment of Alopecia (Hair Loss) (not according to the claims)

Twenty-nine (29) year-old male had exhibited signs of hair loss for over 2 years. Processed microvascular tissue was reconstituted in 4cc of sterile water for injection. Multiple injections of 0.1-0.2 cc aliquots were made through a 30g needle, starting in the center of the 2" diameter treatment area then radiating out.

Two (2) months after treatment the patient was seen for a follow-up visit. The treating physician noticed fine hair growth at the injection site.

### Example 13: Treatment of Bursitis (not according to the claims)

A 59-year-old female complained of pain in her left hip that had persisted for 5 years making sleep, gardening, walking or running uncomfortable even with NSAIDS. Physical examination and X-rays showed moderate arthritis in the hip joint, but most of the pain was associated with inflamed bursa and radiated along the Sartorius muscle. Microvascular tissue was reconstituted with 4 ml Marcaine that was injected under ultrasound guidance in 12 sites in and under the inflamed bursa along the pain track. Patient reported diminished pain over the next two days, one painful twinge on day 3, another painful twinge on day 13 (over use), and no twinges since. Five months later, she reported that she does not even think about her hip any longer.

## Claims

1. A composition comprising dried and sterilized multipotent cells wherein the cells have been subjected to one or more processing treatments, which result in enhanced bioactivity, wherein the one or more processing treatments comprise irradiation at a minimum threshold dose of 40kGy.

2. The composition of claim 1 wherein the one or more processing treatments comprise ultrasonic agitation and/or fluid shear force.

3. The composition of claim 1 or claim 2 wherein the one or more processing treatments comprise storage for about one day at 46°C.

4. The composition of any of claims 1 or 2 wherein the one or more processing treatments comprise storage for about two years at room temperature.

5. The composition of claims 1-4 further wherein the cells have decreased ability to proliferate.

6. The composition of any of claims 1-5 wherein the multipotent cells were live, cultured cells.

7. The composition of any of claims 1-6 wherein bioactivity is measured as one or more parameters selected from the group consisting of cell proliferation, cell migration, anti-inflammation, vascularization, tissue healing and pain relief, wherein optionally the multipotent cells subjected to one or more processing treatments have greater bioactivity than the equivalent dose of live untreated multipotent cells.

8. A composition comprising processed microvascular tissue wherein the tissue has been subjected to one or more processing treatments, which result in enhanced bioactivity, wherein the one or more processing treatments comprise irradiation at a minimum threshold dose of 40kGy.

9. The composition of claim 8 wherein the one or more processing treatments comprise ultrasonic agitation and/or fluid shear force.

10. The composition of claim 8 or claim 9 wherein the one or more processing treatments comprise storage for about one day at 46°C.

11. The composition of any of claims 8 or 9 wherein the one or more processing treatments comprise storage about two years at room temperature.

12. A composition comprising non-viable dried and sterilized multipotent cells or processed microvascular tissue, wherein the composition has been subjected to one or more treatments that enhance bioactivity, for use in a method of treating or preventing an injury or disease in a mammal, or for use in a method for relieving tissue pain associated with a medical condition, or for use in a method for wound healing, or for use in a method of preventing or treating scars, wherein the one or more processing treatments comprise irradiation at a minimum threshold dose of 40kGy; and optionally storage for about one day at 46°C or storage for about two years at room temperature.

13. The composition for the use of claim 12, wherein the one or more processing treatments comprise fluid shear force and/or ultrasound agitation, and/or wherein the medical condition is selected from the group consisting of arthritis, plantar fasciitis, bursitis, temporomandibular joint pain, osteoarthritis, hair loss, fat grafting, cleft palate, osteomyelitis, peripheral neuropathy, chronic wounds, fractures, and tendon or ligament disorders.

14. The composition for the use of claims 12 or 13 wherein the composition is a topical powder, a topical cake, topical cream, dried film, or an injection.

15. Method of enhancing the bioactivity of a composition, comprising: providing a composition comprising dried and sterilized multipotent cells or processed microvascular tissue; subjecting the composition to irradiation at a minimum threshold dose of 40kGy; optionally storing the composition for about one day at 46°C or storage for about two years at room temperature; and optionally subjecting the composition to ultrasonic agitation and/or fluid shear force.

## Patentansprüche

1. Zusammensetzung, umfassend getrocknete und sterilisierte multipotente Zellen, wobei die Zellen einer oder mehreren Verarbeitungsbehandlungen unterzogen wurden, die zu einer erhöhten Bioaktivität führen, wobei die eine oder mehreren Verarbeitungsbehandlungen Bestrahlung mit einer Mindestschwellendosis von 40 kGy umfassen.

2. Zusammensetzung nach Anspruch 1, wobei die eine oder mehreren Verarbeitungsbehandlungen Rühren mit Ultraschall und/oder Flüssigkeitsscherkraft umfassen.

3. Zusammensetzung nach Anspruch 1 oder Anspruch 2, wobei die eine oder mehreren Verarbeitungsbehandlungen eine Lagerung für etwa einen Tag bei 46 °C umfassen.

4. Zusammensetzung nach einem der Ansprüche 1 oder 2, wobei die eine oder mehreren Verarbeitungsbehandlungen eine Lagerung für etwa zwei Jahren bei Raumtemperatur umfassen.

5. Zusammensetzung nach Anspruch 1-4, wobei die Zellen ferner eine verringerte Fähigkeit zur Proliferation aufweisen.

6. Zusammensetzung nach einem der Ansprüche 1-5, wobei die multipotenten Zellen lebende, kultivierte Zellen waren.

7. Zusammensetzung nach einem der Ansprüche 1-6, wobei die Bioaktivität als ein oder mehrere Parameter gemessen wird, die aus der Gruppe bestehend aus Zellproliferation, Zellmigration, Entzündungshemmung, Vaskularisierung, Gewebeheilung und Schmerzlinderung ausgewählt sind, wobei die multipotenten Zellen, die einer oder mehreren Verarbeitungsbehandlungen unterzogen wurden, gegebenenfalls eine größere Bioaktivität aufweisen als die äquivalente Dosis lebender, unbehandelter multipotenter Zellen.

8. Zusammensetzung, umfassend verarbeitetes mikrovaskuläres Gewebe, wobei das Gewebe einer oder mehreren Verarbeitungsbehandlungen unterzogen wurde, die zu einer erhöhten Bioaktivität führen, wobei die eine oder mehreren Verarbeitungsbehandlungen Bestrahlung mit einer Mindestschwellendosis von 40 kGy umfassen.

9. Zusammensetzung nach Anspruch 8, wobei die eine oder mehreren Verarbeitungsbehandlungen Rühren mit Ultraschall und/oder Flüssigkeitsscherkraft umfassen.

10. Zusammensetzung nach Anspruch 8 oder Anspruch 9, wobei die eine oder mehreren Verarbeitungsbehandlungen eine Lagerung für etwa einen Tag bei 46 °C umfassen.

11. Zusammensetzung nach einem der Ansprüche 8 oder 9, wobei die eine oder mehreren Verarbeitungsbehandlungen eine Lagerung für etwa zwei Jahren bei Raumtemperatur umfassen.

12. Zusammensetzung, umfassend nicht lebensfähige getrocknete und sterilisierte multipotente Zellen oder verarbeitetes mikrovaskuläres Gewebe, wobei die Zusammensetzung einer oder mehreren Behandlungen unterzogen wurde, die die Bioaktivität erhöhen, zur Verwendung in einem Verfahren zum Behandeln oder Vorbeugen einer Verletzung oder Erkrankung bei einem Säugetier, oder zur Verwendung in einem Verfahren zum Lindern von Gewebeschmerzen, die mit einem medizinischen Zustand verbunden sind, oder zur Verwendung in einem Verfahren zur Wundheilung, oder zur Verwendung in einem Verfahren zum Vorbeugen oder Behandeln von Narben, wobei die eine oder mehreren Verarbeitungsbehandlungen Bestrahlung mit einer Mindestschwellendosis von 40 kGy umfassen; und gegebenenfalls Lagerung für etwa einen Tag bei 46 °C oder Lagerung für etwa zwei Jahre bei Raumtemperatur.

13. Zusammensetzung zur Verwendung nach Anspruch 12, wobei die eine oder mehreren Verarbeitungsbehandlungen Flüssigkeitsscherkraft und/oder Rühren mit Ultraschall umfassen, und/oder wobei der medizinische Zustand aus der Gruppe bestehend aus Arthritis, Plantarfasziitis, Bursitis, Kiefergelenkschmerzen, Osteoarthritis, Haarausfall, Fetttransplantation, Gaumenspalte, Osteomyelitis, peripherer Neuropathie, chronischen Wunden, Frakturen und Sehnen- oder Bänderstörungen ausgewählt ist.

14. Zusammensetzung zur Verwendung nach Anspruch 12 oder 13, wobei die Zusammensetzung ein topisches Pulver, ein topischer Kuchen, eine topische Creme, ein getrockneter Film oder eine Injektion ist.

15. Verfahren zum Erhöhen der Bioaktivität einer Zusammensetzung, umfassend: Bereitstellen einer Zusammensetzung, die getrocknete und sterilisierte multipotente Zellen oder verarbeitetes mikrovaskuläres Gewebe umfasst; Unterziehen der Zusammensetzung einer Bestrahlung mit einer Mindestschwellendosis von 40 kGy; gegebenenfalls Lagern der Zusammensetzung für etwa einen Tag bei 46 °C oder Lagern für etwa zwei Jahre bei Raumtemperatur; und gegebenenfalls Unterziehen der Zusammensetzung einem Rühren mit Ultraschall und/oder Flüssigkeitsscherkraft.

## Revendications

1. Composition comprenant des cellules multipotentes séchées et stérilisées, dans laquelle les cellules ont été soumises à un ou plusieurs traitements de transformation, qui résultent en une bioactivité améliorée, dans laquelle les un ou plusieurs traitements de transformation comprennent une irradiation à une dose seuil minimale de 40 kGy.

2. Composition selon la revendication 1, dans laquelle les un ou plusieurs traitements de transformation comprennent une agitation par ultrasons et/ou une force de cisaillement de fluide.

3. Composition selon la revendication 1 ou la revendication 2, dans laquelle les un ou plusieurs traitements de transformation comprennent un stockage pendant environ un jour à 46 °C.

4. Composition selon l'une quelconque des revendications 1 et 2, dans laquelle les un ou plusieurs traitements de transformation comprennent un stockage pendant environ deux ans à température ambiante.

5. Composition selon les revendications 1 à 4, dans laquelle en outre les cellules ont une capacité réduite à proliférer.

6. Composition selon l'une quelconque des revendications 1 à 5, dans laquelle les cellules multipotentes étaient des cellules vivantes, cultivées.

7. Composition selon l'une quelconque des revendications 1 à 6, dans laquelle la bioactivité est mesurée comme un ou plusieurs paramètres choisis dans le groupe constitué par la prolifération cellulaire, la migration cellulaire, l'anti-inflammation, la vascularisation, la cicatrisation des tissus et le soulagement de la douleur, dans laquelle éventuellement les cellules multipotentes soumises à un ou plusieurs traitements de transformation ont une bioactivité supérieure à la dose équivalente de cellules multipotentes vivantes non traitées.

8. Composition comprenant un tissu microvasculaire traité, dans laquelle le tissu a été soumis à un ou plusieurs traitements de transformation, qui résultent en une bioactivité améliorée, dans laquelle les un ou plusieurs traitements de transformation comprennent une irradiation à une dose seuil minimale de 40 kGy.

9. Composition selon la revendication 8, dans laquelle les un ou plusieurs traitements de transformation comprennent une agitation par ultrasons et/ou une force de cisaillement de fluide.

10. Composition selon la revendication 8 ou la revendication 9, dans laquelle les un ou plusieurs traitements de transformation comprennent un stockage pendant environ un jour à 46 °C.

11. Composition selon l'une quelconque des revendications 8 et 9, dans laquelle les un ou plusieurs traitements de transformation comprennent un stockage pendant environ deux ans à température ambiante.

12. Composition comprenant des cellules multipotentes non viables séchées et stérilisées ou un tissu microvasculaire traité, dans laquelle la composition a été soumise à un ou plusieurs traitements qui améliorent la bioactivité, pour utilisation dans un procédé de traitement ou de prévention d'une blessure ou d'une maladie chez un mammifère, ou pour utilisation dans un procédé de soulagement de la douleur tissulaire associée à un état médical, ou pour utilisation dans un procédé de cicatrisation, ou pour utilisation dans un procédé de prévention ou de traitement des cicatrices, dans laquelle les un ou plusieurs traitements de transformation comprennent une irradiation à une dose seuil minimale de 40 kGy ; et éventuellement un stockage pendant environ un jour à 46 °C ou un stockage pendant environ deux ans à température ambiante.

13. Composition pour l'utilisation selon la revendication 12, dans laquelle les un ou plusieurs traitements de transformation comprennent une force de cisaillement de fluide et/ou une agitation par ultrasons, et/ou dans laquelle l'état médical est choisi dans le groupe constitué par l'arthrite, la fasciite plantaire, la bursite, la douleur de l'articulation temporomandibulaire, l'arthrose, la perte de cheveux, la greffe de graisse, la fente palatine, l'ostéomyélite, la neuropathie périphérique, les plaies chroniques, les fractures et les troubles des tendons ou des ligaments.

14. Composition pour l'utilisation selon la revendication 12 ou 13, dans laquelle la composition est une poudre topique, une poudre tassée topique, une crème topique, un film séché ou une injection.

15. Procédé d'amélioration de la bioactivité d'une composition, comprenant : la fourniture d'une composition comprenant des cellules multipotentes séchées et stérilisées ou un tissu microvasculaire traité ; la soumission de la composition à une irradiation à une dose seuil minimale de 40 kGy ; éventuellement le stockage de la composition pendant environ un jour à 46 °C ou le stockage pendant environ deux ans à température ambiante ; et éventuellement la soumission de la composition à une agitation par ultrasons et/ou à une force de cisaillement fluide.
